# EUROPEAN PATENT APPLICATION

(11) **EP 4 772 030 A1**
(43) Date of publication of application: **08.07.2026**
(21) Application number: 24860124.7
(22) Date of filing: 28.06.2024
(51) Int. Cl.: A24B 15/16, A24B 13/00, A24F 23/02, A24B 15/24, A24B 15/30, A24B 15/32, A24B 15/42, A24B 15/28, A24B 15/38, A61K 31/465

(54) **NICOTINE POUCH FILLER AND MANUFACTURING METHOD THEREFOR**

(30) Priority: 31.08.2023 KR 20230115724
(71) Applicant: KT & G Corporation, Daejeon 34337 (KR)
(72) Inventor: JEOUNG, Eun Mi, Daejeon 34128 (KR); KI, Sung Jong, Daejeon 34128 (KR); CHA, Sung Je, Daejeon 34128 (KR); SHIN, Jun Won, Daejeon 34128 (KR)
(74) Representative: Ter Meer Steinmeister & Partner
(86) International application number: PCT/KR2024/009047
(87) International publication number: WO 2025/048200

(57) **Abstract**

The present invention relates to a nicotine pouch filler comprising: nicotine; a release control excipient including at least one selected from the group consisting of cellulose and a sugar alcohol; a binder, a pH adjuster; and a flavoring, wherein the content of nicotine is 2.5-10 wt% with respect to the total solid content of the nicotine pouch filler, and the content of the release control excipient is 5.5-36 times that of nicotine.

## Description

### TECHNICAL FIELD

One or more embodiments relate to a nicotine pouch filler and a method of preparing the nicotine pouch filler.

### BACKGROUND ART

Nicotine pouches are one type of smoking article and are used in the mouth of a user to provide nicotine satisfaction to the user. For example, nicotine pouches may be used by a user placing the pouch between the upper or lower gums and lips and holding it there for a limited period of time.

A nicotine pouch includes a filler packaged in a packaging material. The packaging material holds the nicotine pouch filler in place and allows saliva to pass through the nicotine pouch filler, and a flavor and nicotine released from the nicotine pouch filler diffuse into the user's mouth.

Therefore, there is a need for a nicotine pouch in which most or all of the nicotine may be released in an appropriate amount from the nicotine pouch filler and nicotine may be effectively absorbed.

### DISCLOSURE OF THE INVENTION

### TECHNICAL GOALS

Embodiments provide a nicotine pouch filler with improved elution rate and absorption rate of nicotine.

However, goals to be achieved are not limited to those described above, and other goals not mentioned above are clearly understood by one of ordinary skill in the art from the following description.

### TECHNICAL SOLUTIONS

According to an aspect, there is provided a nicotine pouch filler including nicotine, a release control excipient including at least one selected from a group consisting of cellulose and sugar alcohol, a binder, a pH adjuster, and a flavoring agent, wherein a content of the nicotine is 2.5 to 10 wt% with respect to a weight of total solid contents of the nicotine pouch filler, and a content of the release control excipient is 5.5 to 36 times the content of the nicotine.

According to another aspect, there is provided a nicotine pouch including a nicotine pouch filler, and a packaging material, wherein the nicotine pouch filler includes nicotine, a release control excipient including at least one selected from a group consisting of cellulose and sugar alcohol, a binder, a pH adjuster, and a flavoring agent, a content of the nicotine is 2.5 to 10 wt% with respect to a weight of total solid contents of the nicotine pouch filler, and a content of the release control excipient is 5.5 to 36 times the content of the nicotine.

According to still another aspect, there is provided a method of preparing a nicotine pouch filler, the method including step S1 of mixing at least one selected from a group consisting of nicotine, a release control excipient, a binder, a pH adjuster, and a flavoring agent, step S2 of spraying a solvent, and step S3 of performing granulation, wherein, in step S1, the release control excipient includes at least one selected from a group consisting of cellulose and sugar alcohol, in step S2, the solvent includes at least one selected from a group consisting of water and alcohol, a content of the solvent is 10 to 60 wt% of a content of the cellulose, and an L/S ratio is 8% to 15%.

### EFFECTS OF THE INVENTION

A nicotine pouch filler may effectively elute nicotine from a nicotine pouch to promote absorption of eluted nicotine, thereby providing the user with satisfaction of smoking.

It should be understood that the effects of the present disclosure are not limited to the above-described effects, but are construed as including all effects that may be inferred from the configurations and features described in the following description or claims of the present disclosure.

### BRIEF DESCRIPTION OF THE DRAWINGS

FIG. 1 is a diagram illustrating a method of preparing a nicotine pouch filler according to an embodiment;
FIG. 2 is a diagram illustrating an elution rate according to a content of microcrystalline cellulose (MCC) of a nicotine pouch filler according to an embodiment; and
FIG. 3 is a diagram illustrating an elution rate according to added sugar alcohol of a nicotine pouch filler according to an embodiment.

### BEST MODE FOR CARRYING OUT THE INVENTION

Hereinafter, embodiments will be described in detail with reference to the accompanying drawings. However, various alterations and modifications may be made to the embodiments and thus, the scope of the disclosure is not limited or restricted to the embodiments. The equivalents should be understood to include all changes, equivalents, and replacements within the idea and the technical scope of the disclosure.

The terminology used herein is for the purpose of describing particular embodiments only and is not to be limiting of the embodiments. The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless disclosed to the contrary, the description of any one embodiment may be applied to other embodiments, and the specific description of the repeated configuration will be omitted.

It will be understood that when a certain part "includes" a certain component, the part does not exclude another component but may further include another component.

According to an embodiment, there is provided a nicotine pouch filler including nicotine, a release control excipient including at least one selected from a group consisting of cellulose and sugar alcohol, a binder, a pH adjuster, and a flavoring agent, wherein a content of the nicotine is 2.5 to 10 wt% with respect to a weight of total solid contents of the nicotine pouch filler, and a content of the release control excipient is 5.5 to 36 times the content of the nicotine.

In the nicotine pouch filler according to an embodiment, the nicotine may include nicotine salt, and the nicotine salt may include at least one selected from a group consisting of nicotine salt benzoate, nicotine salt tartrate, nicotine salt malate, and nicotine salt salicylate.

In the nicotine pouch filler according to an embodiment, the content of the nicotine is 2.5 to 10 wt% with respect to the weight of the total solid contents of the nicotine pouch filler. The content of the nicotine may be determined according to a desired strength of the nicotine pouch. When the content of the nicotine is less than the range described above, it is difficult to give the smoker the satisfaction of smoking. On the other hand, when the content of the nicotine exceeds the range described above, nicotine stimulation may be excessive.

The release control excipient adjusts a nicotine elution rate, and may provide a nicotine pouch that may supply a suitable amount of nicotine when using a nicotine pouch. Desirably, a content of the release control excipient may be 5.5 to 36 times the content of nicotine. When the content of the release control excipient is beyond the range described above, the pouch filler may dissolve quickly or the nicotine elution rate may be delayed. Also, nuclei of particles of the nicotine pouch filler may not be sufficiently formed, severe agglomeration or crushing of a powder may occur, making it difficult to prepare the nicotine pouch filler. In addition, nicotine stimulation may be excessive or too weak.

In the nicotine pouch filler according to an embodiment, the release control excipient may include cellulose, and the nicotine may be contained in the cellulose. That is, cellulose may function as a carrier for nicotine.

In addition, cellulose may function as a diluent with a high water holding capacity to promote the wetting of a mixture in a powder form through swelling and wicking and quickly release saliva and nicotine.

Also, the cellulose may provide usability. When the cellulose is not added, the nicotine pouch filler may come out in a dissolved form.

In the nicotine pouch filler according to an embodiment, the cellulose may include one or more of microcrystalline cellulose (MCC) or methyl cellulose.

In the nicotine pouch filler according to an embodiment, the content of the cellulose may be 5 to 20 wt% with respect to the weight of the total solid contents of the nicotine pouch filler.

When the content of the cellulose is less than the numerical range described above, the nicotine pouch filler may dissolve quickly. On the other hand, when the content of the cellulose exceeds the numerical range described above, the nicotine elution rate may be delayed due to a water-insoluble characteristic of cellulose.

The sugar alcohol is highly soluble in saliva and loosely binds to a solvent than the cellulose. Accordingly, the sugar alcohol may more rapidly release nicotine within the time of use, suppress a bitter taste, and provide a cooling effect when dissolved in the mouth.

In addition, the sugar alcohol may promote nucleation and granule growth in a high-shear wet granulation process of the nicotine pouch filler.

In the nicotine pouch filler according to an embodiment, heat of dissolution of the sugar alcohol may be -190 J/g to -10 J/g. For example, the heat of dissolution of the sugar alcohols may be -185 J/g to -20 J/g, -125 J/g to -20 J/g, -40 J/g to -23 J/g, or -23 J/g. When the heat of dissolution of the sugar alcohol exceeds the numerical range described above, it is difficult to provide a sufficient cooling experience to the user.

In the nicotine pouch filler according to an embodiment, a solubility of the sugar alcohol may be 10 g/100g to 80 g/100g, desirably 20 g/100g to 65 g/100g, and more desirably 60 g/100g to 65 g/100g at 20°C. When the solubility of the sugar alcohol is below the numerical range described above, it may not be sufficiently dissolved in the user's mouth. On the other hand, when the solubility of the sugar alcohol exceeds the numerical range described above, nicotine may release excessively.

In the nicotine pouch filler according to an embodiment, the sugar alcohol may include at least one selected from a group consisting of erythritol, xylitol, mannitol, sorbitol, maltitol, and isomalt. However, the sugar alcohol is not limited to the examples listed above.

In the nicotine pouch filler according to an embodiment, a content of the sugar alcohol may be 50 to 70 wt%, desirably, 60 to 70 wt% with respect to the weight of the total solid contents of the nicotine pouch filler. When the content of the sugar alcohol is below the numerical range described above, nuclei of particles of the nicotine pouch filler may not be sufficiently formed. Meanwhile, when the content of the sugar alcohol exceeds the numerical range described above, severe agglomeration or crushing of a powder may occur, making it difficult to prepare the nicotine pouch filler.

A type and a content of the binder may affect the formation of a size distribution of nicotine pouch filler particles.

In the nicotine pouch filler according to an embodiment, the binder may include at least one selected from a group consisting of Povidone K-25, hydroxypropyl cellulose (HPC), low-substituted hydroxypropyl cellulose (L-HPC), hydroxypropylmethyl cellulose (HPMC), and carboxymethylcellulose (CMC). Desirably, the binder may be HPC. Meanwhile, the L-HPC may refer to that a content of a hydroxypropyl group is 5.0% to 16.0%.

In the nicotine pouch filler according to an embodiment, a content of the binder may be 10 wt% or less, desirably, 8 to 15 wt% with respect to the weight of the total solid contents of the nicotine pouch filler.

When the type and the content of the binder are outside the range described above, it may be difficult to secure a required particle size and may have a negative effect on initial elution.

The pH adjuster may adjust the pH of the nicotine pouch filler to a pH with which nicotine may be absorbed such that nicotine may be converted to a free-based nicotine form that may be absorbed quickly when the nicotine pouch is inserted into the mouth.

The free-based nicotine is a nonionic substance in which hydrogen ions have been removed from nicotine salt, and may be absorbed quickly through mucous membranes and have a strong impact on consumers.

In the nicotine pouch filler according to an embodiment, the pH of the nicotine pouch filler may be 8.3 to 8.5. When the pH is 8.02 or more, nicotine may be converted into the free-based nicotine form, which may be quickly absorbed through the mucous membrane. Therefore, when the pH of the nicotine pouch filler satisfies the numerical range described above, the pH may be 8.02 or more when mixed with saliva (pH 6.5 to 7.7), and thus, nicotine may be easily absorbed and have a strong impact on consumers.

In the nicotine pouch filler according to an embodiment, the pH adjuster may be at least one selected from a group consisting of sodium bicarbonate and sodium carbonate.

At this time, the pH adjuster may include sodium bicarbonate and sodium carbonate, and a weight ratio of the sodium bicarbonate to sodium carbonate may be 7:3 to 6:4, desirably 6:4. When a higher proportion of sodium bicarbonate is added than the ratio described above (a ratio with a relatively lower proportion of sodium carbonate), the pH may be lowered and nicotine may not be sufficiently absorbed. On the other hand, when a lower proportion of sodium bicarbonate is added than the ratio described above (a ratio with a relatively higher proportion of sodium carbonate), the pH may become higher than the desired range and may not be suitable for elution in the mouth.

The flavoring agent may provide a taste to satisfy the smoker's preference.

In the nicotine pouch filler according to an embodiment, the flavoring agent may include at least one selected from a group consisting of menthol, licorice, sucrose, fructose syrup, isosweetener, cocoa, lavender, cinnamon, cardamom, celery, fenugreek, cascarilla, sandalwood, bergamot, geranium, honey essence, rose oil, vanilla, lemon oil, orange oil, mint oil, caraway, cognac, jasmine, chamomile, cinnamon, ylang ylang, sage, spearmint, peppermint, ginger, coriander, and coffee. However, the flavoring agent is not limited to the examples described above.

The nicotine pouch filler according to an embodiment is not limited to including the components described above, and may optionally additionally include a humectant, an antioxidant, a preservatives, or the like.

For example, the humectant may include one or more of glycerin and propylene glycol.

The antioxidant may include, for example, one or more of ascorbyl palmitate and sodium ascorbate.

The preservative may include, for example, xylitol, or the like.

The nicotine pouch filler according to an embodiment may have nicotine elution performance of 10% to 25% within 4 minutes, and desirably nicotine elution performance of 15% to 25%, and 18% to 22%.

The nicotine pouch filler according to an embodiment may have nicotine elution performance of 50% to 90% within 20 minutes, and desirably nicotine elution performance of 70% to 80%.

When the nicotine elution performance is less than the numerical range described above, it may be difficult to give the user the satisfaction of smoking. On the other hand, when the nicotine elution performance exceeds the numerical range described above, it may give stimulation to the user.

According to an embodiment, there is provided a nicotine pouch including the nicotine pouch filler described above, and a packaging material.

The packaging material may package a predetermined amount of the nicotine pouch filler. The packaging material may be packaged so that the nicotine pouch filler inside does not leak out during transportation and use after being packaged, and may be prepared so that smokers do not feel a foreign body sensation in their mouth when using it.

The packaging material may be safe for the human body, hold saliva well, have a certain strength to prevent tearing, and have sealing properties.

A material of the packaging material may be a cellulose fiber, a thermoplastic material fiber, or a combination thereof. For example, the cellulose may include rayon, and the thermoplastic material fiber may be nylon, polyethylene, polylactic acid, polypropylene, polyethersulfone, or the like, but is not limited to the examples described above. The material of the packaging material may further include a binder.

The packaging material may be a nonwoven type or woven type material.

A method of preparing the packaging material may include a process of forming a web and a process of bonding the web.

The process of forming the web may be a dry-laid method, a wet-laid method, or a spinning method, and the dry method may be a carding method or an air ray method. The wet-laid method may be a general paper preparing method or a preparing method obtained by partially modifying it. The spinning method may be a spun-bonded or melt-blown method.

Meanwhile, the process of bonding the web may include a mechanical bonding method, a chemical bonding method, or a thermal bonding method. The mechanical bonding method may include a needle-punching method or a spun-lace method. The chemical bonding method may include a polymer dispersion method or a polymer solution method. The thermal bonding method may include a calendering method, a hot air method, an ultrasonic method, or the like.

Desirably, the packaging material may be prepared by the spun-lace method and/or the wet-laid method. The spun-lace method may include forming a web in a form of a sheet with a predetermined thickness with crumpled fiber raw material, forming a material of a packaging material by spraying moisture such that fibers are tangled, and drying. The wet-laid method may include preparing a dispersion by dispersing a fiber raw material in water, forming a material of a packaging material by moving the dispersion to a paper machine, and dehydrating and drying. However, the spun-lace method and the wet-laid method are not limited to including the processes described above.

The packaging material may have a porosity to release the nicotine pouch filler filled inside the packaging material.

The packaging material may be packaged in three-sided packaging or back packaging, and sealing of the packaging material may be performed by ultrasonic sealing or heat sealing. The packaging material may be formed with a longitudinal seal formed along a horizontal direction of the packaging material and a cross seal formed along a vertical direction of the packaging material, and the longitudinal seal and/or cross seal may be sealed by lip sealing or fin sealing.

The packaging material may be classified into original, slim, super slim, and mini forms according to sizes and weights thereof. The packaging material may have an appropriate internal volume according to a capacity of the nicotine pouch filler to provide appropriate hardness and comfortable use.

The finally prepared nicotine pouch may be wrapped and distributed.

According to an embodiment, there is provided a method of preparing a nicotine pouch filler. The method of preparing the nicotine pouch filler may include step S1 of mixing at least one selected from a group consisting of nicotine, a release control excipient, a binder, a pH adjuster, and a flavoring agent, step S2 of spraying a solvent, and step S3 of performing granulation, wherein, in step S1, the release control excipient includes at least one selected from a group consisting of cellulose and sugar alcohol, in step S2, the solvent includes at least one selected from a group consisting of water and alcohol, a content of the solvent is 10 to 60 wt% of a content of the cellulose, and an L/S ratio is 8% to 15%.

Hereinafter, each step will be described in detail.

Step S1 is step of mixing at least one selected from a group consisting of nicotine, a release control excipient, a binder, a pH adjuster, and a flavoring agent. At this time, the components may be prepared in a powder form and may be uniformly mixed.

The nicotine may include nicotine salt, and the type of nicotine salt is the same as the examples described above.

The release control excipient may include at least one selected from a group consisting of cellulose and sugar alcohol. The sugar alcohol in the release control excipient may promote nucleation and granule growth in the granulation step.

The type of the binder is the same as the examples described above. A method of adding the binder may particularly affect the granulation step. The binder may be added by a wet binder adding method or a dry binder adding method, desirably by a dry binder adding method. When the binder is added by the dry binder adding method, it is easy to form particles having a desired size, and a uniform particle size distribution may be obtained, thereby facilitating production.

The types of the pH adjuster and the flavoring agent are the same as the examples described above.

Step S2 is step of spraying a solvent. At this time, the solvent may be sprayed onto and mixed with a powder mixture prepared in step S1.

At this time, the solvent may be at least one of water or alcohol, desirably alcohol.

A content of the solvent may be 10 to 60 wt%, desirably 40 to 50 wt% of the content of the cellulose. The amount of solvent to be added may be represented by an L/S ratio. The L/S ratio is represented as a total weight of the solvent/a total weight of the solid powder mixture, and the total weight of the solid powder mixture may be a total weight of at least one selected form a group consisting of the nicotine, the release control excipient, the binder, the pH adjuster, and the flavoring agent added in step S1. The L/S ratio may be 8% to 15%, desirably 10% to 12%. When the L/S ratio satisfies the numerical range described above, the size is uniform, fine particles with a size of less than 100 µm are generated only in small amounts or not at all, and particles with a porous structure may be formed, which is advantageous in nicotine release. When the L/S ratio is less than the numerical range described above, granules do not form and may exist in a fine powder form. On the other hand, when the L/S ratio exceeds the numerical range described above, irregular particles are formed, the particle size range becomes small, and agglomeration may occur during mixing.

Step S3 is step of performing granulation. At this time, after stirring the powder mixture prepared in step S2 and a solvent for 5 to 30 minutes, the clumps of particles agglomerated during the stirring process may be loosened and separated into individual particles.

The granulated nicotine pouch filler particles may be evenly distributed in size. For example, the nicotine pouch filler may include 70% or more of particles with a size of 200 µm or more, and less than 30% of particles with a size of 100 µm or more and less than 200 µm.

Step S3 may further include step S4 of performing low-temperature drying after step S3. At this time, the nicotine pouch filler granulated in step S3 may be dried at 50°C for 1 to 3 hours, and in the drying step, the clumps of the agglomerated particles may be loosened and separated into individual particles, and then particles having a desired size may be selected.

Hereinafter, the present disclosure will be described in more detail with reference to examples, however, the present disclosure is not limited to the examples below.

### 1. Example 1: Nicotine elution rate according to content of MCC

### Preparation Example 1

A nicotine pouch including a nicotine pouch filler was prepared. At this time, the nicotine pouch filler included 4.5 wt% of nicotine, 20 wt% of MCC, 50 to 60 wt% of sugar alcohol, 10 wt% of a pH adjuster, and 8 wt% of a binder.

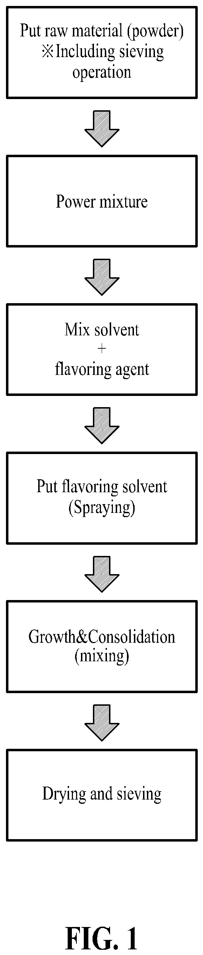

A specific preparation method is as shown in FIG. 1.

### Comparative Example 1

A nicotine pouch was prepared in the same manner as in Preparation Example 1, except that the content of MCC was 25 wt%.

### Comparative Example 2

A nicotine pouch was prepared in the same manner as in Preparation Example 1, except that the content of MCC was 40 wt%.

### Comparative Example 3

A nicotine pouch was prepared in the same manner as in Preparation Example 1, except that the content of MCC was 55 wt%.

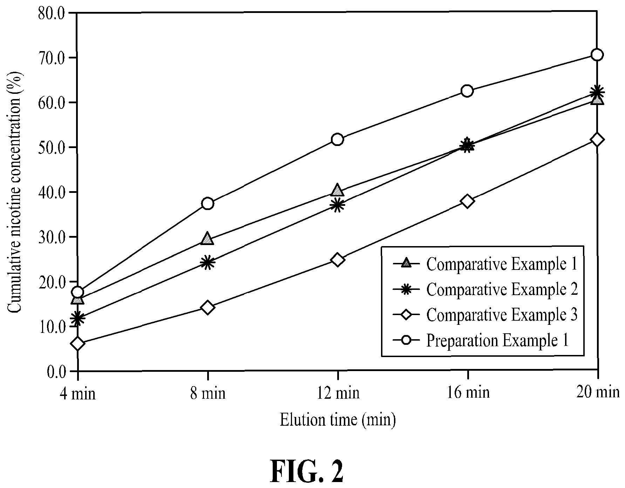

Nicotine elution rates of Preparation Example 1 and Comparative Examples 1 to 3 were analyzed. The nicotine elution rate was evaluated by measuring a content of released nicotine after leaving Preparation Examples and Comparative Examples in 1 liter of a phosphate buffer maintained at a temperature of 37°C and a pH of 7.4 for 1 minute using a USP paddle device. The results thereof are shown in FIG. 2 and Table 1.

**[Table 1]**

| Classification | Preparation Example 1 | Comparative Example 1 | Comparative Example 2 | Comparative Example 3 |
|---|---|---|---|---|
| MCC content (wt%) | 20 | 25 | 40 | 55 |
| Elution rate after first 4 minutes (%) | 17.5 | 16.1 | 11.7 | 6.1 |
| Elution rate after 20 minutes (%) | 70.1 | 59.9 | 61.7 | 51.2 |

Preparation Example 1 showed a highest elution rate, with an elution rate of 17.5% after the first 4 minutes. However, as the content of MCC increased, the elution rate tended to decrease after the first 4 minutes due to the water-insoluble characteristic of MCC (Comparative Examples 1 to 3).

### 2. Example 2: Nicotine elution rate according to addition of sugar alcohol

### Preparation Example 2

A nicotine pouch including a nicotine pouch filler was prepared. The nicotine pouch filler included nicotine, MCC, and maltitol, a content of maltitol was 70 wt% with respect to a weight of total solid contents, and a content of MCC was 5 wt% with respect to the weight of total solid contents.

### Preparation Example 3

A nicotine pouch was prepared in the same manner as in Preparation Example 2, except that the content of maltitol was 55 wt% with respect to the weight of total solid contents, and accordingly, the content of MCC was 20 wt% with respect to the weight of total solid contents.

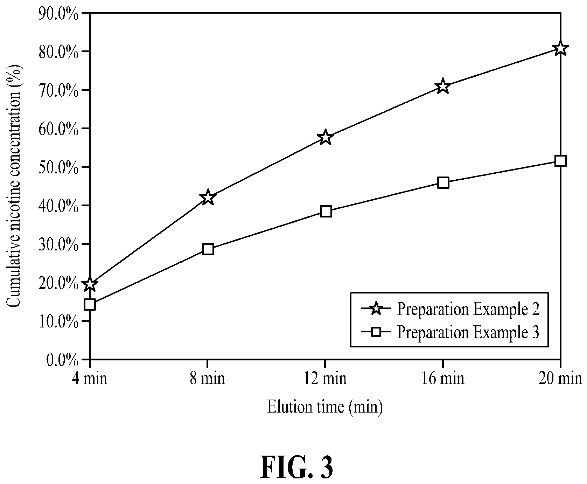

Nicotine elution rates of Preparation Examples 2 and 3 were analyzed. The nicotine elution rate was evaluated in the same manner as in the Example 1 described above. The results thereof are shown in FIG. 3.

As a result, Preparation Example 2 showed a nicotine elution rate of 20% after the first 4 minutes and 80% after 20 minutes, and Preparation Example 3 showed a nicotine elution rate of 14% after the first 4 minutes and 51.6% after 20 minutes. The fact that Preparation Example 2 showed a higher elution rate compared to Preparation Example 3 is determined to be due to the higher content of sugar alcohol.

### 3. Example 3: Distribution of particle size and nicotine elution rate according to type of binder

### Preparation Example 4

A nicotine pouch including a nicotine pouch filler was prepared. The nicotine pouch filler included nicotine, MCC, maltitol, a binder, a pH adjuster, and a flavoring agent, the binder was HPC, and a content of the binder was 8 wt% with respect to the weight of total solid contents.

The nicotine pouch filler was prepared using a dry binder addition method. After first mixing nicotine, MCC, maltitol, a binder, a pH adjuster, and a flavoring agent, a solvent, that is water alone, water + alcohol, or alcohol alone, was sprayed, and a granulation process was performed to prepare the nicotine pouch filler.

### Preparation Example 5

A nicotine pouch filler was prepared in the same manner as in Preparation Example 4, except that Povidone K-25 was used as a binder and alcohol was applied as a solvent.

### Preparation Example 6

A nicotine pouch filler was prepared in the same manner as in Preparation Example 4, except that L-HPC was used as a binder.

Nicotine elution rates of Preparation Examples 2 and 4 to 6 above were analyzed. The nicotine elution rate was evaluated in the same manner as in the Example 1 described above, and the results thereof were shown in Table 2.

**[Table 2]**

| Classification | | Preparation Example 2 | Preparation Example 4 | Preparation Example 5 | Preparation Example 6 |
|---|---|---|---|---|---|
| Elution rate after first 4 minutes (%) | | 20 | 17.5 | 15.3 | 13.8 |
| Elution rate after 20 minutes (%) | | 80 | 70.1 | 61.9 | 55.3 |
| Distribution of particle size (%) | Size of 200 µm or more | 70 | 66.7 | 50.5 | 61.8 |
| | Size of 50 µm or less | None | None | 2.2 | 1.6 |

In Preparation Examples 2 and 4, 66.7% or more of nicotine pouch filler particles with a size of 200 µm or more were distributed, and there were no nicotine pouch filler particles with a size of 50 µm or less. Preparation Examples 2 and 4 had higher nicotine elution rates not only initially but also after 20 minutes compared to Preparation Examples 5 and 6.

### 4. Example 4: Free-based nicotine generation rate according to pH range and proportion of pH adjuster

### Preparation Example 7

A nicotine pouch including a nicotine pouch filler was prepared. The nicotine pouch filler included a pH adjuster, and sodium bicarbonate and sodium carbonate were used as the pH adjuster in a ratio of 6:4.

### Preparation Example 8

A nicotine pouch filler was prepared in the same manner as in Preparation Example 7, except that sodium bicarbonate and sodium carbonate were used as the pH adjuster in a ratio of 7:3.

### Comparative Example 4

A nicotine pouch filler was prepared in the same manner as in Preparation Example 7, except that sodium bicarbonate and sodium carbonate were used as the pH adjuster in a ratio of 8:2.

A pH and a free-based nicotine generation rate of the nicotine pouch fillers of Preparation Examples 7 and 8 and Comparative Example 4 were investigated, and the results thereof are shown in Table 3.

**[Table 3]**

| Classification | pH | Free-based nicotine generation rate (%) |
|---|---|---|
| Preparation Example 7 | 8.8 | 85% to 90% |
| Preparation Example 8 | 8.4 | 76% |
| Comparative Example 4 | 7.6 | 24% |

Unlike Comparative Example 4, Preparation Examples 7 and 8 showed results in which the pH of saliva was 8.8 and 8.4, respectively, and the free-based nicotine generation rate was 76% or more. It is confirmed that, when saliva with a pH of 6.5 to 7.7 is introduced, the pH becomes 8.02 or more, and thus, more free-based nicotine is generated.

From the above experimental examples, it is expected that using the nicotine pouch filler described in the claims may easily make nicotine to be eluted and promote absorption of the eluted nicotine.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if described components are combined in a different manner, and/or replaced or supplemented by other components or their equivalents.

Therefore, other implementations, other embodiments, and equivalents to the claims are also within the scope of the following claims.

## Claims

1. A nicotine pouch filler comprising:
nicotine;
a release control excipient comprising at least one selected from a group consisting of cellulose and sugar alcohol;
a binder;
a pH adjuster; and
a flavoring agent,
wherein a content of the nicotine is 2.5 to 10 wt% with respect to a weight of total solid contents of the nicotine pouch filler, and
wherein a content of the release control excipient is 5.5 to 36 times the content of the nicotine.

2. The nicotine pouch filler of claim 1, wherein
the nicotine comprises nicotine salt, and
the nicotine salt comprises at least one selected from a group consisting of nicotine salt benzoate, nicotine salt tartrate, nicotine salt malate, and nicotine salt salicylate.

3. The nicotine pouch filler of claim 1, wherein
the release control excipient comprises cellulose, and
the nicotine is contained in the cellulose.

4. The nicotine pouch filler of claim 1, wherein the cellulose comprises one or more of microcrystalline cellulose and methyl cellulose.

5. The nicotine pouch filler of claim 1, wherein a content of the cellulose is 5 to 20 wt% with respect to a weight of total solid contents of the nicotine pouch filler.

6. The nicotine pouch filler of claim 1, wherein the sugar alcohol comprises at least one selected from a group consisting of erythritol, xylitol, mannitol, sorbitol, maltitol, and isomaltol.

7. The nicotine pouch filler of claim 1, wherein a content of the sugar alcohol is 50 to 70 wt% with respect to a weight of total solid contents of the nicotine pouch filler.

8. The nicotine pouch filler of claim 1, wherein
the binder comprises at least one selected from a group consisting of Povidone K-25, hydroxypropyl cellulose, low-substituted hydroxypropyl cellulose, hydroxypropylmethyl cellulose (HPMC), and carboxymethylcellulose (CMC), and
a content of the binder is 8 to 10 wt% with respect to a weight of total solid contents of the nicotine pouch filler.

9. The nicotine pouch filler of claim 1, wherein the pH adjuster comprises one or more of sodium bicarbonate and sodium carbonate.

10. The nicotine pouch filler of claim 9, wherein
the pH adjuster comprises sodium bicarbonate and sodium carbonate, and
a weight ratio of the sodium bicarbonate and the sodium carbonate is 7:3 to 6:4.

11. The nicotine pouch filler of claim 1, wherein a pH of the nicotine pouch filler is 8.3 to 8.5.

12. The nicotine pouch filler of claim 1, wherein the nicotine pouch filler has nicotine elution performance of 15% to 25% within 4 minutes, and nicotine elution performance of 70% to 80% within 20 minutes.

13. A nicotine pouch comprising:
a nicotine pouch filler; and
a packaging material,
wherein the nicotine pouch filler comprises:
nicotine;
a release control excipient comprising at least one selected from a group consisting of cellulose and sugar alcohol;
a binder;
a pH adjuster; and
a flavoring agent,
wherein a content of the nicotine is 2.5 to 10 wt% with respect to a weight of total solid contents of the nicotine pouch filler, and
wherein a content of the release control excipient is 5.5 to 36 times the content of the nicotine.

14. A method of preparing a nicotine pouch filler, the method comprising:
step S1 of mixing at least one selected from a group consisting of nicotine, a release control excipient, a binder, a pH adjuster, and a flavoring agent;
step S2 of spraying a solvent; and
step S3 of performing granulation,
wherein, in step S1, the release control excipient comprises at least one selected from a group consisting of cellulose and sugar alcohol,
wherein, in step S2, the solvent comprises at least one selected from a group consisting of water and alcohol,
wherein a content of the solvent is 10 to 60 wt% of a content of the cellulose, and
wherein an L/S ratio is 8% to 15%.
